Europäisches Patentamt

European Patent Office

Office européen des brevets

(19)

(11) Numéro de publication: **0 139 555**
**B1**

(12) **FASCICULE DE BREVET EUROPÉEN**

(45) Date de publication du fascicule du brevet:
14.01.87

(51) Int. Cl.⁴: **C 07 K 7/08,** A 61 K 37/02,
G 01 N 33/569

(21) Numéro de dépôt: **84401720.2**

(22) Date de dépôt: **27.08.84**

(54) **Nouveau peptide de synthèse, son procédé de préparation et médicaments le contenant.**

(30) Priorité: **29.08.83 FR 8313828**

(43) Date de publication de la demande:
**02.05.85 Bulletin 85/18**

(45) Mention de la délivrance du brevet:
**14.01.87 Bulletin 87/3**

(84) Etats contractants désignés:
**AT BE CH DE FR GB IT LI LU NL SE**

(56) Documents cité:
**FR-A-2 395 030**
**US-A-4 392 997**

(73) Titulaire: **INSTITUT PASTEUR Fondation reconnue d'utilité publique, 28 rue du Docteur Roux, F-75724 Paris Cédex 15 (FR)**

(72) Inventeur: **Dodin, André, 29 Bld Edgar Quinet, F-75014 Paris (FR)**
Inventeur: **Siffert, Odile, 17 rue des Missionnaires, F-78000 Versailles (FR)**
Inventeur: **Le Thuillier, Georgette, née Tchernoff, 12 Rue Jean Moulin, F-91420 Morangis (FR)**
Inventeur: **Allard, Patrice, 60 rue Piat, F-75020 Paris (FR)**

(74) Mandataire: **Ores, Irène, CABINET ORES 6, Avenue de Messine, F-75008 Paris (FR)**

EP 0 139 555 B1

LIBER, STOCKHOLM 1987

## Description

La présente invention est relative à un nouveau peptide qui comprend une séquence d'acides aminés reproduisant plus particulièrement le site d'interaction entre la sous-unité A et la sous-unité B de la toxine cholérique.

Comme on le sait [ Cf. J. HOLMGREN, NATURE, 292, 30 Juillet 1981, p. 413-417 ] la diarrhée aqueuse caractéristique du choléra qui, si elle n'est pas enrayée, conduit à la déshydratation, à l'acidose métabolique du malade et à la mort, est due à la toxine cholérique secrétée par le Vibrio cholerae. On connait bien le mécanisme d'action de la toxine cholérique, qui se fixe sur des récepteurs qui se trouvent sur les cellules muqueuses et stimule l'activité adényl cyclase intestinale, qui a pour effet d'augmenter le taux d'A.M.P. cyclique présente dans les cellules de l'intestin grêle; cette dernière provoque la diarrhée et la perte de fluide par sortie massive d'eau et inhbition de l'absorption du chlorure de sodium par les villosités intestinales et par la stimulation de la secrétion active de chlorure par les cellules cryptiques.

L'on sait également que la toxine cholérique est une protéine constituée de deux fragments polypeptidiques: la sous-unité B, comprenant elle-même cinq unités identiques, et la sous-unité A [Cf. R.A. FINKELSTEIN, M.K. LARUE et J.J. LOSPALLUTO, INFECT. IMMUN., 6, 1972, pp 934 ]. Il est admis [Cf. J. HOLMGREN, Loc. cité ] que l'action nocive de la toxine cholérique se traduit par les faits suivants: fixation de la sous-unité B de la toxine sur le récepteur GMI de la membrane de l'entérocyte duodénal, suivie d'une augmentation du taux d'A.M.P. cyclique due à une activation irréversible de l'adényl cyclase [Cf. D.M. GILL, ADV. CYCLIC NUCLEOTIDE RES., 8, 1977, pp. 85 ] ce qui laisserait penser que la symptomatologie du choléra découlerait de l'introduction de la sous-unité A dans la cellule.

Cette sous-unité A présente un caractère hydrophobe qui est mis en évidence lorsqu'elle est fractionnée de la toxine cholérique (CT), par réduction du pont disulfure, pour donner les sous-unités $\alpha$ et $\gamma$ non-identiques. Selon W.H.J. WARD, P. BRITTON et S. VAN HEYNINGEN, (BIOCHEM. J., 199, 1981, pp 457), le fragment $\alpha$ n'aurait pas de régions hydrophobes et, par contre, le fragment $\gamma$ aurait un caractère amphiphile, les régions hydrophobes pouvant être masquées dans la sous-unité A intacte. Enfin, ce serait grâce au fragment $\gamma$, conducteur, que le fragment $\alpha$ pénétrerait à travers la membrane de la cellule. C'est pourquoi, il a paru intéressant et utile aux Inventeurs de rechercher dans la structure de la sous-unité A les séquences d'acides aminés responsables des activités biologiques de la toxine cholérique.

Par ailleurs, les travaux de E.K. SPICER, W.H. KAVANAUGH, W.S. DALLAS, S. FALKOW, W.H. KONIGSBERG et D.E. SCHAFER, dont il est rendu compte dans PROC. NATL. ACAD. SCI, U.S.A., 78, Janvier 1981, p. 50-54 et ceux de W.S. DALLAS et S. FALKOW (NATURE, 288, 4 Décembre 1980, P. 499-501) ont mis en évidence l'existence de similitudes de structure primaire entre la toxine cholérique (CT) et l'entérotoxine thermolabile d'Escherichia coli (LT) responsable des gastro-entérites infectieuses: la toxine cholérique CT et la toxine thermolabile LT d'Escherichia coli sont des entérotoxines similaires du point de vue fonctionnel, structural et immunologique. Elle provoquent toutes deux l'élévation des taux d'A.M.P. cyclique dans les cellules épithéliales des intestins en catalysant l'ADP-ribosylation NAD-dépendante des protéines membranaires. Elles se composent toutes deux de deux sous-unités dissemblables, à savoir la sous-unité A de l'entérotoxine qui a une activité enzymatique et est le composant activateur de l'adényl cyclase et la sous-unité B qui reconnaît les composants membranaires et fixe l'holotoxine à la cellule cible, juxtaposant la sous-unité A avec ses substrats. Il a été démontré que les récepteurs membranaires de la sous-unité B de la toxine cholérique et de la toxine d'Escherichia coli sont similaires mais non identiques, et que le monosialosylganglioside GM1 est le récepteur de la CT et constitue probablement une partie du récepteur de la LT. Il a été démontré [ cf. CL GYLES et D.A. BARNUM, J. INFECT. DIS., 120, p. 419-426 (1978) ] que la LT et la CT sont similaires du point de vue immunologique et qu'aussi bien leurs sous-unités A que leurs sous-unités B présentent des déterminants antigéniques communs [.cf. J.D.CLEMENTS et R.A. FINKELSTEIN, INFECT. IMMUN., 21, p. 1036-1039 (1978) ]. La structure primaire des sous-unités B de la LT et de la CT a été déterminée et a fait apparaître qu'elles présentent une séquence homologue d'acides aminés. Les travaux de SPICER et Al et de DALLAS et Al cités plus haut, ont montré qu'il existe également des similitudes de structure primaire et de propriétés immunologiques entre les sous-unités A de la CT et de la LT et bien que les séquences en acides aminés de ces deux sous-unités ne soient que partiellement identifiées à ce jour, cependant l'on sait d'après les travaux de DUFFY et LAI [ BIOCHEM. BIOPHYS. RES. COMM., 91, 1979, pp. 1005 ] que la sous-unité A de la CT a un poids moléculaire de 29500, dont 24000 pour la chaine $\alpha$ et 5500 pour la chaîne $\gamma$ dont on connaît la composition en acides aminés, laquelle comporte 46 acides aminés dont 11 communs avec la chaîne $\gamma$ de la LT.

En se fondant sur l'hypothèse selon laquelle le site d'interaction entre la sous-unité A et la sous-unité B de la CT serait localisé dans la chaîne $\gamma$ et plus particuliérement dans la séquence correspondant aux acides aminés portant les numéros 10 à 24 à partir du N terminal de cette dernière, et en se fondant sur la similitude fonctionnelle, structurelle et immologique entre la LT et la CT, les Inventeurs ont considéré que s'ils parvenaient à synthétiser un polypeptide présentant cette séquence 10-24, ils pourraient obtenir un agent de protection à la fois contre le choléra et contre les gastro-entérites infectieuses.

La présente invention a en consécuence pour but de pourroir à un peptide reproduisant au moins 10 acides aminés de la chaîne $\gamma$ de la sous-unité A de la toxine cholérique communs avec la chaîne $\gamma$ de la toxine LT de E. coli ou à un peptide susceptible d'induire (seul ou couplé à une molécule immunogène telle qu'une protéine comme la sérumalbumine bovine par example) après injection à un animal, des anticorps reconnaissant la séquence commune telle que définie ci-dessus, ou son équivalent, puisqu'il est, en effet, connu que certains

2

acides aminés peuvent être remplacés par d'autres sans que la structure secondaire soit notablement modifiée.

De manière préférée, la présente invention a pour but de pourvoir à un pentadécapeptide reproduisant la séquence 10-24 de la chaîne γ de la sous-unité A de toxine cholérique et présentant 5 acides aminés communs avec la chaîne γ de la sous-unité A de la toxine d'Escherichia coli, susceptible de servir d'agent de protection, et notamment de vaccin, au moins contre le choléra et de préférence à la fois contre le choléra et contre les gastro-entérites infectieuses. Ce pentadécapeptide peut être reproduit, conformément à l'invention, par synthèse chimique ou par tout autre moyen, tel que l'utilisation de bactéries transformées par des vecteurs porteurs d'une séquence de nucléotides correspondants aux acides aminés.

La présente invention a pour objet un peptide caractérisé en ce qu'il reproduit au moins 5 acides aminés de la chaîne γ de la sous-unité A de la toxine cholérique communs avec la chaîne γ de la toxine LT d'Escherichia coli.

Selon un mode de réalisation préféré de l'invention, celle-ci à pour objet un pentadécapeptide de synthèse caractérisé en ce qu'il répond à la formule I ci-après: (I)

| Gln | Ser | Leu | Gly | Val | Lys | Phe | Leu | Asp | Glu | Tyr | Gln | Ser | Lys | Val |
|-----|-----|-----|-----|-----|-----|-----|-----|-----|-----|-----|-----|-----|-----|-----|
| 10  | 11  | 12  | 13  | 14  | 15  | 16  | 17  | 18  | 19  | 20  | 21  | 22  | 23  | 24  |

laquelle formule correspond à la séquence 10-24 de la chaine γ de la sous-unité A de la toxine cholérique et présente 5 des 11 acides aminés communs avec la chaine γ de la sous-unité A de la toxine thermolabile LT d'Escherichia coli.

L'étude de la structure secondaire de la chaîne γ de la sous-unité A de la CT, faite par la méthode de CHOU et FASMAN /ADVANCES IN ENZYMOL., 47, 1978, pp. 45] a fait apparaître qu'il existe deux régions ayant une structure en hélice; la première se situe entre les positions 5 et 12 et la seconde entre les positions 15 et 24. Ces deux régions sont hydrophiles [d'après la méthode de HOPP et WOODS (PROC. NATL. ACAD. SCI. U.S.A., 78, 1981, p. 3824] et représentent une partie de la séquence ayant des acides aminés communs avec la LT.

La présente invention a également pour objet un procédé de synthèse du peptide comprenant au moins 5 des 11 acides aminés communs à la chaîne γ de la sous-unité A de la toxine cholérique et à la chaîne γ de la toxine LT d'Escherichia coli, qui est caractérisé en ce que ledit peptide est synthétisé par construction de la chaîne peptidique pas-à-pas, en partant de l'extrémité C terminale de ladite chaîne, par fixation successive des acides aminés qui constituent celle-ci.

Selon un mode de réalisation préféré du procédé objet de la présente invention, appliqué à la synthèse du pentadécapeptide de formule I défini ci-dessus, celui-ci est synthétisé par construction de la chaîne peptidique pas-à-pas, en partant de l'extrémité C terminale, c'est-àdire en partant de la valine, par fixation successive des acides aminés qui constituent la chaîne peptidique.

Selon un mode de mise en oeuvre préféré du procédé de synthèse conforme à la présente invention, celui-ci est réalisé en phase solide, en présence d'un agent de couplage approprié.

Selon une modalité préférée de ce mode de mise en oeuvre, la synthèse en phase solide est réalisée en utilisant un support solide constitué par une résine, de préférence une résine chlorométhylée ou une résine phénolique, notamment.

Selon un autre mode de mise en oeuvre préféré du procédé de synthèse conforme à l'invention, la fonction α-aminée des acides aminés successivement fixés pour former la chaîne peptidique recherchée, est protégée temporairement par un groupe t-Butoxycarbonyle(Boc).

Selon encore un autre mode de mise en oeuvre préféré du procédé de synthése conforme à l'invention, les fonctions latérales des acides aminés successivement fixés pour former la chaîne peptidique recherchée, sont protégées jusqu'à la fin de la synthése par des groupes benzyles.

Selon une disposition avantageuse de ce mode de mise en oeuvre, les groupes carboxyles des acides aspartique et glutamique sont protégés par des groupes ester benzylique.

Selon une autre disposition avantageuse de ce mode de mise en oeuvre, le groupe hydroxyle de la sérine est protégé par un groupe benzyléther.

Selon encore une autre disposition avantageuse de ce mode de mise en oeuvre, l'amine e de la lysine est protégée par un groupe carbobenzyloxy (CBZ).

Selon un mode de mise en oeuvre préféré du procédé de synthése conforme à l'invention, l'agent de couplage mis en oeuvre pour réaliser la construction pas à pas de la chaîne peptidique, est un mélange équimoléculaire de dicyclohexylcarbodiimide et de 1-hydroxybenzotriazole dans un solvant approprié, tel que, notamment, le diméthylformamide ou le chloruré de méthylène.

Selon un autre mode de mise en oeuvre préféré du procédé de synthése conforme à l'invention, le groupe protecteur Boc de la fonction α-aminée des acides aminés successivement fixés, est enlevé par acidolyse.

Selon encore un autre mode de mise en oeuvre préféré du procédé de synthése conforme à l'invention, les groupements protecteurs des fonctions latérales des acides aminés successivement fixés pour former la chaîne peptidique recherchée, sont éliminés et la fonction C terminale est libérée, simultanément une fois la synthèse terminée, par action d'un acide fort, tel que, notamment, l'acide fluorhydrique liquide.

Selon un autre mode de mise en oeuvre préféré du procédé de synthèse conforme à l'invention le peptide libre obtenu après élimination des groupes protecteurs, est purifié par passage sur au moins une colonne de tamis moléculaire approprié, puis purification par chromatographie en phase liquide à haute performance.

La présente invention a en outre pour objet un agent de protection contre le choléra et/ou les gastro-entérites infectieuses, pris dans le groupe qui comprend les sérums et les vaccins administrables par voiesparentêrale sous-cutanée, cutanée ou orale, caractérisé en ce qu'il comprend le pentadécapeptide de synthése de formule I défini plus haut.

Selon un mode de réalisation avantageux de l'agent de protection conforme à la présente invention, celui-ci comprend ledit pentadécapeptide de synthése de formule associé à une sous-unité B de toxine cholérique et/ou à une sous-unité B de toxine d'Escherichia coli.

La présente invention a pour objet un médicament nouveau caractérisé en ce qu'il comprend, en tant que constituant actif un peptide conforme à l'invention et notamment, le pentadécapeptide de formule I défini plus haut, ledit médicament nouveau étant apte à traiter les maladies liées à une activation du système adénylcyclase - AMP cyclique.

Conformément à la présente invention, le peptide de synthèse peut être utilisé en tant qu'agent de diagnostic du choléra et/ou des gastro-entérites.

Selon une modalité de mise en oeuvre avantageuse de cette application, l'agent de diagnostic comprenant ledit peptide de synthèse est avantageusement constitué par un sérum.

Conformément à l'invention, un sérum obtenu à partir du peptide de synthèse conforme à l'invention est apte à être utilisé en tant qu'agent de diagnostic de la présence d'Escherichia coli toxinogénes (responsables de gastroentérites infectieuses).

Egalement conformément à l'invention, un tel sérum utilisable en tant qu'agent de diagnostic de germes responsables de gastro-entérites infectieuses, est constitué par du sérum provenant de lapins préalablement traités par le peptide de synthèse conforme à l'invention.

Le sérum obtenu conformément à la présente invention est utilisé en tant qu'agent de diagnostic dans un test de détection de la présence de germes responsables de gastroentérites, qui consiste à mettre ledit agent de diagnostic en contact avec un milieu biologique (souche du germe isolée sur gélose, selles, urines ou sang du sujet que l'on suppose contaminé ou eau contaminée), pendant une durée appropriée, de l'ordre de 16 heures à 24 heures, mais de préférence pendant 18 heures, au bout de laquelle l'apparition d'une bande de précipité antigène-anticorps met en évidence la présence desdits germes.

Ce test de diagnostic est d'une grande sensibilité, puisqu'il permet de détecter la présence de germes pathogènes chez un sujet porteur d'une faible quantité de ces germes, dans de très petites quantités de milieux biologiques, ce qui vient s'ajouter au fait que ce test ne requiert qu'un temps très bref, puisqu'il suffit de 18 heures en moyenne pour que se révèle la bande de précipité antigène-anticorps qui indique la présence de germes pathogènes, alors que les tests de diagnostic pour la détection de la présence d'Escherichia coli toxinogènes connus dans l'Art antérieur nécessitent 5 jours et sont très coûteux par rapport au test de diagnostic objet de la présente invention.

Pour la réalisation de ce test de diagnostic il est prévu, conformément à la présente invention, un coffret de diagnostic prêt à l'emploi qui comprend une quantité appropriée du peptide conforme à l'invention ainsi qu'une quantité appropriée d'anticorps anti-peptide conforme à l'invention, comme standard de référence, le standard de référence étant avantageusement constitué par une quantité appropriée d'anticorps anti-peptide 10-24.

La synthèse du pentadécapeptide de formule I, conforme à la présente invention, sera décrite ci-après dans l'exemple qui va suivre, qui est donné uniquement à titre d'illustration et n'a aucun caractère limitatif.

La méthode de synthèse mise en oeuvre est une méthode en phase solide pour la synthèse de peptides dérivée de la méthode de R.B. MERRIFIELD [ J. AMER. CHEM. SOC., 85, 1963, p. 2149 ].

La construction de la chaîne peptidique est réalisée en partant de l'extrémité C terminale qui, dans le cas présent, est la valine, par fixation successive des acides aminés, selon la méthode pas à pas.

On part de 1,5 g de résine chlorométhylée "Bio Beads S - XI" (de BIO-RAD) d'une capacité de 1,34 milliéquivalents/ gramme de grosseur des grains: 200-400 mesh, sur laquelle on fixe la valine sous forme de Boc-valine par gramme de résine; on obtient 1,85 g de résine-valine substituée à 0,46 mM/g, soit 0,69 mM pour 1,85 g (Test de GISIN). Le couplage des acides aminés successifs est réalisé avec un excès, de préférence le double, par rapport à la substitution, soit:

Boc-acide amine: 1,4 mmole, en utilisant un agent de couplage:

Hydroxybenzotriazole: 1,4 mmole

Dicyclohexylcarbodiimide: 1,4 mmole dans du chlorure de méthylène et/ou du diméthyl-formamide comme solvants.

La fonction $\alpha$-aminée des acides aminés est protégée temporairement, au moment de la fixation de l'acide aminé, par un groupe Boc, tandis que les fonctions latérales sont protégées par le groupe ester benzylique pour les fonctions acide, par le groupe benzyl éther pour les fonctions alcool et par le groupe carbobenzyloxy pour l'amine $\varepsilon$ de la lysine.

Les contrôles de couplage et de déblocage sont effectués par le test à la ninhydrine (Test de KAISER).

Le groupement Boc est éliminé par acidolyse à l'aide d'acide trifluoracétique à 30 % dans le dichlorométhane, et les autres groupes, y compris la fonction C terminale, sont libérés, en fin de synthèse, par action de l'acide fluorhydrique liquide.

Le peptide libre est purifié par passages successifs sur colonnes de tamis moléculaire, à savoir:

"ULTROGEL ACA 201". Elution par l'acide acétique 0,1 M (Marque commerciale appartenant à L.K.B.)

"BIOGEL P4". Elution par l'acide acétique 1 M (Marque commerciale de BIORAD)

"BIOGEL P4". Elution par l'acide acétique 0,1 M (Marque commerciale de BIORAD)

Les analyses en acides aminés des différentes fractions ont permis d'isoler le peptide recherché, qui est finalement purifié par chromatographie en phase liquide à haute performance (CLHP) sur une colonne phase inverse dans les conditions suivantes: colonne "LICHROSORB" RB 18 Marque commerciale déposée par MERCK (5 µm) 250 mm X 4 mm. Eluant A: acétonitrile; Eluant B: $KH_2PO_4$, $5 \times 10^{-3}$M (pH = 6,0; débit: 1,5 ml/min). Gradient linéaire de 20 à 80 % de A en 30 minutes. Détection UV à 220 nm, 0,02 unité de densité optique pleine échelle. La pureté du produit (TR: 7,18 min) est de 94 %, déduction faite de la surface des pics dus au solvant (T.R. < 2,50 minutes).

## PROPRIETES BIOLOGIQUES DE LA SEQUENCE SYNTHETIQUE 10-24

Les activités biologiques de la toxine cholérique complète sont extrémement diverses, elles se résument dans les activités enzymatiques ayant comme médiateur 1'A.M.P. cyclique. Le caractère immunogène du peptide 10-24 de synthése a été mis en évidence en procédant comme suit: **MATERIEL ET METHODE:**

Des lapins ont été immunisés avec le peptide synthétique conforme à l'invention, selon la technique de OUDIN (lére injection, toxine synthétique, 100 mcg, + adjuvant de FREUND par voie intradermique, tous les 4 jours ensuite, une injection intramusculaire puis sous-cutanée et intra-veineuse sans adjuvant). Le sérum est recueilli 15 jours apres.

Double diffusion en gélose: le sérum a été étudié par double diffusion en gélose selon la technique d'OUCHTERLONY.

Les tests biologiques ont été réalisés sur l'anse duodénale de souris $C^3H$ se¹on la technique de FUJITA et FINKELSTEIN, avec 10 mcg de la séquence synthétique comparativement à 10 mcg de toxine cholérique purifiée. **RESULTATS:**

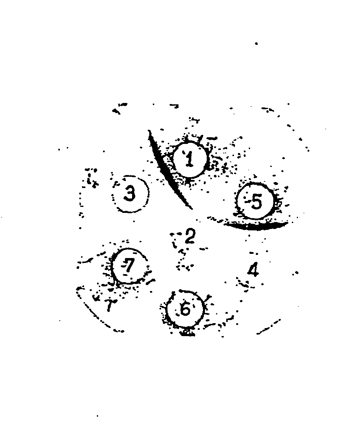

In vitro: Les résultats obtenus in vitro sont illustrés à la figure unique annexée qui représente le diagramme d'OUCHTERLONY de double diffusion en gélose révélant les bandes de précipitation de la toxine et des anticorps de sérum de lapin sensibilisé avec le pentadécapeptide conforme à l'invention, reproduisant la séquence 10-24 de la chaîne γ. Selon ce diagramme, le sérum antitoxine cholérique contenu dans les puits 1.7.5. ne reconnaît pas le fragment synthétique de trop faible poids moléculaire contenu dans le puits 2 (puits 1 contre 2 et 5 contre 2), mais reconnaît la toxine complète contenue dans les puits 3-4 (puits 1 contre 3 et 5 contre 4). Les sérums contenus dans les puits 6-7 obtenus contre le fragment de synthèse ne donnent aucune bande de précipitation contre le fragment synthétique (7 contre 2), par contre, un des sérums (7) reconnaît la séquence des acides aminés dans la toxine complète (3) (7 contre 3). Une plaque de double diffusion en gélose à disposition identique avec les sérums 1,5,6 et 7 avant immunisation est totalement négative.

In vivo: La recherche de l'activité du peptide synthétique conforme à l'invention,qui reproduit un fragment de la sous-unité A, a été effectuée dans l'anse in testinale de souris $C^3H$. Les résultats comparatifs (mesure du poids par cm d'intestin par rapport à un témoin animal pesant de 60 à 65 mg) sont donnés en milligrammes par centimètre d'intestin et représentent la moyenne de 3 souris par expérience:

a) après introduction de 10 mcg de toxine cholérique (Sigma lot 122 F 0239) dans l'anse intestinale après 18 heures: 126,7 mg.

b) après introduction de 10 mcg de sous-unité B: 67 mg (Sigma lot 12 F 0503);

c) après introduction de 10 mcg de sous-unité A: 62 mg (Sigma lot 122 F 0240)

d) après introduction de 10 mcg de sous-unité B (même référence) puis après 10 minutes 100 mcg de sous-unité.A (même référence): 116,6 mg

e) après introduction de 100 mcg de sous-unité B (même référence) puis après 10 minutes: 10 mcg de toxine cholérique (même référence). 126 7 mg

f) après introduction de 100 mcg de fragment synthétique de sous-unité A: 61 mg

g) après introduction de 10 mcg de sous-unité B (même référence) puis après 10 minutes 100 mcg de fragment synthétique de sous-unité A: 63,2 mg

h) après introduction de 100 mcg de sous-unité B + 100 mcg de fragment synthétique de sous-unité A, puis après 10 minutes 10 mcg de toxine cholérique (méme référence): 67 mg

Ces résultats font apparaître que le pentadécapeptide de synthèse, conforme à l'invention, a provoqué chez le lapin des anticorps capables de reconnaître, dans la toxine cholérique complète la séquence d'A.A. correspondant au constituant de synthèse.

En association avec la sous-unité B; le pentadécapeptide conforme à l'invention, a inhibé la sortie de l'eau hors des tissus par action de la toxine cholérique du commerce.

Le pentadécapeptide conforme à l'invention, a joué le rôle de "leurre" vis-à-vis de la toxine.

Etant donné le degré important de similitude entre les sous-unités A de la toxine cholérique et de la toxine thermolabile d'Escherichia coli, ce rôle de "leurre" existe également vis-à-vis des Escherichia coli toxinogènes et permet d'envisager à la fois une approche médicamenteuse du traitement de ces deux affections et une approche en tant qu'agent de diagnostic.

Le peptide conforme à l'invention, peut être utilisé sous une forme pharmaceutiquement acceptable,telle que solution, poudre, etc... ou également absorbé ou couplé de manière non covalente à un support approprié,afin de permettre sa libération de manière lente et continue chez le patient.

Outre le fait que le pentadécapeptide de synthèse conforme à la présente invention permet de disposer d'un agent de protection contre le choléra ou contre les gastro-entérites infectieuses, ou à la fois contre l'une et l'autre de ces maladies, il présente un intérêt significatif sur le plan physiologique: les activateurs de l'A.M.P. cyclique sont, en effet, peu mombreux, en sorte qu'il permet d'élucider les mécanismes d'action des activités biologiques liées à la stimulation de la chaine de l'A.M.P. cyclique et d'envisager des traitements thérapeutiques des troubles liés à une activation du système adénylcyclase-A.M.P. cyclique et éventuellement par couplage ou association avec un composant actif tel qu'hormone ou tout autre médiateur biologique.

Outre son utilisation en tant que médicament suivant la double approche qui vient d'être explicitée dans ce qui précède, il s'est avéré que les sérums obtenus par injection du peptide conforme à la présente invention à des lapins permettent de réaliser un excellent diagnostic différentiel des Escherichia coli toxinogènes par rapport aux Escherichia coli entéroinvasives et aux Escherichia coli pathogènes nontoxinogènes. On a décrit ci-après en premier lieu un procédé de préparation du sérum propre à être utilisé dans le test de diagnostic conforme à l'invention, et en second lieu les modalités de la réalisation d'un test de diagnostic utilisant ledit sérum dans différents milieux biologiques.

## PREPARATION D'UN AGENT DE DIAGNOSTIC CONFORME A L'INVENTION

On part du pentadécapeptide synthétique dont la préparation a été décrite plus haut; cette toxine synthétique cristallisée est tout d'abord dissoute en eau physiologique tamponnée à raison de 1 mg[m1.

La solution ainsi préparée est injectée comme suit à des lapins blancs pesant 1,8 à 2 kg, rasés sur une surface 20 cm de chaque côté de la colonne vertébrale:

1ère injection: 1 ml de toxine synthétique (1 mg/ml) + 1 ml d'adjuvant complet de Freund mélangé à 37°C.

On effectue dix injections intradermiques de 0,10 ml de chaque côté de la colonne vertébrale.

2ème injection: après 5 jours, on injecte 1 ml de toxine synthétique par voie intramusculaire profonde.

3ème injection: après 5 jours, on injecte 1 ml de toxine synthétique par voie sous-cutanée.

4ème injection: après 5 jours, on injecte 1 ml de toxine synthétique par voie intraveineuse.

Quinze jours après la quatrième injection, les lapins sont sacrifiés. Le sérum recueilli est titré et réparti en ampoules.

## TEST DE DIAGNOSTIC

Il est réalisé:

- soit sur la souche de E. coli isolée sur gélose de 18 heures,
- soit sur la selle ou sur l'eau contaminée dans laquelle on recherche la présence d'E. coli.

A) Sur la souche isolée

1. Au centre d'une boîte de Petri (diamètre 5 cm) renfermant 10 ml de milieu de Muller-Hinton, on fait une culture (en pastille) d'environ 1 cm.

2. On laisse à l'étuve à 30°C pendant 18 heures.

3. On creuse à 8 mm de la culture initiale de la souche un puits de 8 mm dont on colmate le fond par une goutte de gélose.

4. On place dans le puits le sérum antitoxine synthétique.

5. On prélève à l'ose une parcelle de la culture initiale de la souche isolée pour isolement ultérieur.

6. On lyse la culture en déposant sur la pastille (= zone de culture de la souche) deux gouttes de toluène.

7. On laisse en contact à la température du laboratoire pendant 18 heures. La présence de toxine LT d'E. coli est mise en évidence par l'apparition d'une bande de précipité antigène-anticorps à 1 mm de la pastille.

8. Les plaques de gélose sont ensuite lavées en eau physiologique, séchées entre deux ronds de papier filtre et colorées à l'Amidoschwarz.

B) Dans l'eau

1. Dans un litre d'eau à étudier, on ajoute 0,20 ml de billes de gel magnétique chargées en anticorps anti E. coli totaux telles que celles mises en oeuvre dans la Demande de Brevet français n° 82 20632 du 9 Décembre 1982; on laisse en contact une heure à 37°, sur agitation si possible.

2. On prélève les billes avec un bâton aimanté, comme décrit dans la Demande de Brevet français précitée ou à l'aide du dispositif selon la Demande de Brevet français n° 83 17166 du 27 Octobre 1983, on les dépose au centre d'une boîte de petri contenant du milieu Muller-Hinton.

La suite des opérations est identique à celle décrite aux points A)2. à A)8. ci-dessus.

C) Selles

On émulsionne 1 g environ de selles dans 100 ml d'eau physiologique et on opère commé décrit dans le test B) ci-dessus.

**Revendications** pour les Etats contractants: BE, CH, DE, FR, GB, IT, LI, LU, NL, SE.

1/ Peptide de synthèse caractérisé en ce qu'il reproduit au moins 5 des 11 acides aminés communs à la

chaîne γ de la sous-unité A de la toxine cholérique et à la chaîne γ de la-toxine LT d'Escherichia coli.

2/ Peptide selon la revendication 1, caractérisé en ce qu'il est constitué par un pentadécapeptide qui répond à la formule I ci-après: (I)

```
Gln-Ser-Leu-Gly-Val-Lys-Phe-Leu-Asp-Glu-Tyr-Gln-Ser-Lys-Val
  10   11   12   13   14   15   16   17   18   19   20   21   22   23   24
```

laquelle formule correspond à la séquence 10-24 de la chaîne γ de la sous-unité A de la toxine cholérique et présente 5 des 11 acides aminés communs avec la chaîne γ de la sous-unité A de la toxine thermolabile LT d'Escherichia coli.

3/ Séquence de nucléotides caractérisée en ce qu'elle correspond aux cinq acides aminés minimum du peptide de synthèse selon la revendication 1.

4/ Séquence de nucléotides caractérisée en ce qu'elle correspond aux quinze acides aminés du pentadécapeptide selon la revendication 2.

5/ Procédé de synthèse d'un peptide selon la revendication 1, caractérisé en ce que ledit peptide est synthétisé par construction de la chaîne peptidique pas-à-pas, en partant de l'extrémité C terminale de ladite chaîne, par fixation successive des acides aminés qui constituent celle-ci.

6/ Procédé de synthèse du pentadécapeptide de formule I selon la revendication 2, caractérisé en ce que ledit pentadécapeptide est synthétisé par construction de la chaîne peptidique pas-à-pas, en partant de l'extrémité C terminale, c'est-à-dire en partant de la valine, par fixation successive des acides aminés qui constituent la chaîne peptidique.

7/ Procédé de synthèse selon la revendication 5 ou la revendication 6, caractérisé en ce qu'il est réalisé en phase solide, en présence d'un agent de couplage approprié.

8/ Procédé selon la revendication 7, caractérisé en ce que la synthèse en phase solide est réalisée en utilisant un support solide constitué par une résine, de préférence une résine chlorométhylée ou une résine phénolique, notamment.

9/ Procédé selon l'une quelconque des revendications 5 à 8, caractérisé en ce que la fonction α-aminée des acides aminés successivement fixés pour former la chaîne peptidique recherchée, est protégée temporairement par un groupe t-Butoxycarbonyle (Boc).

10/ procédé selon l'une quelconque des revendications 5 à 9, caractérisé en ce que les fonctions latérales des acides aminés successivement fixés pour former la chaîne peptidique recherchée, sont protégées jusqu'à la fin de la synthèse par des groupes benzyles.

11/ Procédé selon la revendication 10, caractérisé en ce que les groupes carboxyles des acides aspartique et glutamique sont protégés par des groupes ester benzylique.

12/ Procédé selon la revendication 10, caractérisé en ce que le groupe hydroxyle de la sérine est protégé par un groupe benzyléther.

13/ Procédé selon la revendication 10, caractérisé en ce que l'amine ε de la lysine est protégée par un groupe carbobenzyloxy (CBZ).

14/ Procédé selon la revendication 7, caractérisé en ce que l'agent de couplage mis en oeuvre pour réaliser la construction pas à pas de la chaîne peptidique est un mélange équimoléculaire de dicyclohexylcarbodiimide et de 1-hydroxybenzotriazole dans un solvant approprié, tel que, notamment, le diméthylformamide ou le chlorure de méthylène.

15/ Procédé selon la revendication 9, caractérisé en ce que le groupe protecteur Boc de la fonction α-aminée des acides aminés successivement fixés, est enlevé par acidolyse.

16/ Procédé selon l'une quelconque des revendications 10 à 13, caractérisé en ce que les groupements protecteurs des fonctions latérales des acides aminés successivement fixés pour former la chaîne peptidique recherchée, sont éliminés et la fonction C terminale est libérée, simultanément une fois la synthèse terminée, par action d'un acide fort, tel que, notamment, l'acide fluorhydrique liquide.

17/ Procédé selon l'une quelconque des revendications 5 à 16, caractérisé en ce que le peptide libre obtenu après élimination des groupes protecteurs, est purifié par passage sur au moins une colonne de tamis moléculaire approprié, puis purification par chromatographie en phase liquide à haute performance.

18/ Agent de protection contre le choléra et/ou les gastroentérites infectieuses, pris dans le groupe qui comprend les sérums et les vaccins administrables par voies parentèrale, sous-cutanée, cutanée ou orale, caractérisé en ce qu'il comprend un peptide de synthèse selon la revendication 1 ou la revendication 2.

19/ Agent de protection selon la revendication 18, caractérisé en ce qu'il comprend ledit peptide de synthèse associé à une sous-unité B de toxine cholérique et/ou à une sousunité B de toxine d'Escherichia coli.

20/ Médiament nouveau caractérisé en ce qu'il comprend, en étant que constituant actif, un peptide selon la revendication 1 ou la revendication 2, ledit médicament nouveau étant apte à traiter les maladies liées à une activation de 1'A.M.P. cyclique et plus particulièrement liées à une activation du système adénylcyclase - AMP cyclique.

21/ Médicament nouveau pour le traitement du choléra et des gastro-entérites infectieuses, caractérisé en ce qu'il comprend en tant que constituant actif, le peptide de synthèse selon la revendication 1 ou la revendication 2.

22/ Agent de diagnostic du choléra et/ou des gastro-entérites infectieuses, caractérisé en ce qu'il comprend une séquence de nucléotides selon l'une quelconque des revendications 3 et 4.

23/ Agent de diagnostic du choléra et/ou des gastro-entérites infectieuses, caractérisé en ce qu'il comprend un peptide de synthèse selon l'une quelconque des revendications 1 ou 2.

24/ Agent de diagnostic selon la revendication 23, caractérisé en ce qu'il est essentiellement constitué par un sérum préparé à partir du peptide de synthèse selon l'une quelconque des revendications 1 ou 2.

25/ Agent de diagnostic de la présence d'Escherichia coli toxinogènes, caractérisé en ce qu'il est essentiellement constitué par un sérum prélevé chez des lapins préalablement traités par des injections du peptide de synthèse selon l'une quelconque des revendications 1 ou 2.

26/ Test de diagnostic de la présence d'Escherichia coli toxinogènes, caractérisé en ce que l'on met en contact l'agent de diagnostic selon la revendication 25 avec un milieu biologique supposé contaminé par les germes toxinogènes susdits, pendant 16 à 24 heures et de préférence pendant 18 heures, au bout desquelles il apparaît, si le milieu est contaminé, une bande de précipité antigène-anticorps.

27/ Coffret de diagnostic prêt à l'emploi pour la réalisation de tests de diagnostic permettant de détecter la présence de germes pathogènes et notamment pour la détection de la présence d'Escherichia coli toxinogènes, selon la revendication 26, caractérisé en ce qu'il comprend une quantité appropriée d'un agent de diagnostic du choléra et/ou des gastro-entérites infectieuses selon l'une quelconque des revendications 22 à 25 et une quantité appropriée d'un standard de référence constitué par des anticorps antipeptide selon l'une quelconque des revendications 1 ou 2, et notamment par une quantité appropriée d'anticorps antipeptide 10-24.

**Revendications** pour l'Etat contractant: AT

1.- Procédé de synthèse d'un peptide de synthèse qui reproduit au moins 5 des 11 acides aminés communs à la chaîne γ de la sous-unité A de la toxine cholérique et à la chaîne γ de la toxine LT d'Escherichia coli, caractérisé en ce que ledit peptide est synthétisé par construction de la chaîne peptidique pas-à-pas, en partant de l'extrémité C terminale de ladite chaîne, par fixation successive des acides aminés qui constituent celle-ci.

2.- Procédé selon la revendication 1, caractérisé en ce qu'on obtient un pentadécapeptide qui répond à la formule I ci-après

Gln-Ser-Leu-Gly-Val-Lys-Phe-Leu-Asp-Glu-Tyr-Gln-Ser-Lys-Val

10   11   12   13   14   15   16   17   18   19   20   21   22  . 23   24

laquelle formule correspond à la séquence 10-24 de la chaîne γ de la sous-unité A de la toxine cholérique et présente 5 des 11 acides aminés communs avec la chaîne γ de la sousunité A de la toxine thermolabile LT d'Escherichia coli, par construction de la chaîne peptidique pas-à-pas, en partant de l'extrémité C terminale, c'est-à-dire en partant de la valine, par fixation successive des acides aminés qui constituent la chaîne peptidique.

3.- Procédé de synthèse selon la revendication 1 ou 2, caractérisé en ce qu'il est réalisé en phase solide, en présence d'un agent de couplage approprié.

4.- Procédé selon la revendication 3, caractérisé en ce que la synthèse en phase solide est réalisée en utilisant un support solide constitué par une résine, de préférence une résine chlorométhylée ou une résine phénolique, notamment.

5.- Procédé selon l'une quelconque des revendications 1 à 4, caractérisé en ce que la fonction α-aminée des acides aminés successivement fixés pour former la chaîne peptidique recherchée, est protégée temporairement par un groupe t-butoxycarbonyle (Boc).

6.- Procédé selon l'une quelconque des revendications 1 à 5, caractérisé en ce que les fonctions latérales des acides aminés successivement fixés pour former la chaîne peptidique recherchée, sont protégées jusqu'à la fin de la synthèse par des groupes benzyles.

7.- Procédé selon la revendication 6, caractérisé en ce que les groupes carboxyles des acides aspartique et glutamique sont protégés par des groupes ester benzylique.

8.- Procédé selon la revendication 6, caractérisé en ce que le groupe hydroxyle de la sérine est protégé par un groupe benzyléther.

9.- Procédé selon la revendication 6, caractérisé en ce que l'amine ε de la lysine est protégée par un groupe carbobenzyloxy (CBZ).

10.- Procédé selon la revendication 3, caractérisé en ce que l'agent de couplage mis en oeuvre pour réaliser la construction pas-à-pas de la chaîne peptidique est un mélange équimoléculaire de dicyclohexylcarbodiimide et de 1-hydroxybenzotriazole dans un solvant approprié, tel que, notamment, le diméthylformamide ou le chlorure de méthylène.

11.- Procédé selon la revendication 5, caractérisé en ce que le groupe protecteur Boc de la fonction α-aminée des acides aminés successivement fixés, est enlevé par acidolyse.

12.- Procédé selon l'une quelconque des revendications 6 à 9, caractérisé en ce que les groupements protecteurs des fonctions latérales des acides aminés successivement fixés pour former la chaîne peptidique recherchée, sont éliminés et la fonction C terminale est libérée, simultanément une fois la synthèse terminée, par action d'un acide fort, tel que, notamment, l'acide fluorhydrique liquide.

13.- Procédé selon l'une quelconque des revendications 1 à 12, caractérisé en ce que le peptide libre obtenu après élimination des groupes protecteurs, est purifié par passage sur au moins une colonne de tamis moléculaire approprié, puis purification par chromatographie en phase liquide à haute performance.

14.- Procédé selon la revendication 1, caractérisé en ce qu'on obtient cinq acides aminés minimum du peptide de synthèse.

15.- Procédé selon la revendication 2, caractérisé en ce qu'on obtient une séquence de nucléotide correspondant aux quinze acides aminés du pentadécapeptide.

**Patentansprüche** für die Vertragsstaaten: BE, CH, DE, FR, GB, IT, LI, LU, NL, SE.

1. Synthetisches Peptid, dadurch gekennzeichnet, daß es mindestend die der Kette γ der Untereinheit A des cholergen Toxins und der Kette γ des LT-Toxins von Escherichia coli angehören.

2. Peptid nach Anspruch 1, dadurch gekennzeichnet, daß es aus einem Pentadecapeptid besteht, das der nachstehenden Formel I entspricht:

$$\text{Gln-Ser-Leu-Gly-Val-Lys-Phe-Leu-Asp-Glu-Tyr-Gln-Ser-Lys-Val} \quad \text{(I)}$$
$$\;\;10\quad 11\quad 12\quad 13\quad 14\quad 15\quad 16\quad 17\quad 18\quad 19\quad 20\quad 21\quad 22\quad 23\quad 24$$

wobei diese Formel der Sequenz 10-24 der Kette γ der Untereinheit A des cholergen Toxins entspricht und 5 bis 11 Aminosäuren aufweist, die der Kette γ der Untereinheit A des thermolabilen Toxins LT von Escherichia coli angehören.

3. Nukleotidsequenz, dadurch gekennzeichnet, daß sie mindestens 5 Aminosäuren des synthetischen Peptids nach Anspruch 1 entspricht.

4. Nukleotidsequenz, dadurch gekennzeichnet, daß sie 15 Aminosäuren des Pentadecapeptids nach Anspruch 2 entspricht.

5. Verfahren zur Synthese eines Peptids nach Anspruch 1, dadurch gekennzeichnet, daß das Peptid synthetisch hergestellt wird durch schrittweisen Aufbau der Peptidkette, wobei man von dem äußersten Ende C der Kette ausgeht, durch aufeinanderfolgende Fixierung der Aminosäuren, die diese bilden.

6. Syntheseverfahren für das Pentadecapeptid der Formel I nach Anspruch 2, dadurch gekennzeichnet, daß das Pentadecapeptid synthetisch hergestellt wird durch schrittweisen Aufbau der Peptidkette, wobei man von dem äußersten Ende C, d.h. ausgehend von dem Valin, durch aufeinanderfolgende Fixierung der Aminosäuren, die die Peptidkette bilden, ausgeht.

7. Syntheseverfahren nach Anspruch 5 oder Anspruch 6, dadurch gekennzeichnet, daß es in fester Phase in Anwesenheit eines geeigneten Kupplungsmittels durchgeführt wird.

8. Verfahren nach Anspruch 7, dadurch gekennzeichnet, daß die Synthese in fester Phase durchgeführt wird unter Verwendung eines festen Trägers, der aus einem Harz, insbesondere bevorzugt aus einem chlormethylierten Harz oder einem Phenolharz, besteht.

9. Verfahren nach einem der Ansprüche 5 bis 8, dadurch gekennzeichnet, daß die α-Aminfunktion der Aminosäuren, die nacheinander zur Bildung der gewünschten Peptidkette fixiert werden, temporär durch eine t-Butoxycarbonylgruppe (Boc) geschützt wird.

10. Verfahren nach einem der Ansprüche 5 bis 9, dadurch gekennzeichnet, daß die seitlichen Funktionen der nacheinander zur Bildung der gewünschten Peptidkette fixierten Aminosäuren bis zum Ende der Synthese durch Benzylgruppen geschützt werden.

11. Verfahren nach Anspruch 10, dadurch gekennzeichnet, daß die Carboxylgruppen der Asparginsäure und Glutaminsäure durch Benzylestergruppen geschützt werden.

12. Verfahren nach Anspruch 10, dadurch gekennzeichnet, daß die Hydroxylgruppe des Serins durch eine Benzylethergruppe geschützt wird.

13. Verfahren nach Anspruch 10, dadurch gekennzeichnet, daß das ε-Amin des Lysins durch eine Carbobenzyloxigruppe (BZ) geschützt wird.

**0 139 555**

14. Verfahren nach Anspruch 7, dadurch <u>gekennzeichnet</u>, daß das zur Durchführung des schrittweisen Aufbaus der Peptidkette verwendete Kupplungsmittel ein äquimolares Gemisch von Dicyclohexylcarbodiimid und 1-Hydroxybenzotriazol in einem geeigneten Lösungsmittel, wie inbesondere Dimethylformamid oder Methylenchlorid ist.

15. Verfahren nach Anspruch 9, dadurch <u>gekennzeichnet</u>, daß die Schutzgruppe Boc der α-Aminfunktion der nacheinander fixierten Aminosäuren durch Acidolyse abgespalten wird.

16. Verfahren nach einem der Ansprüche 10 bis 13, dadurch <u>gekennzeichnet</u>, daß die Schutzgruppen der seitlichen Funktionen der nacheinander zur Bildung der gewünschten Peptidkette fixierten Aminosäuren entfernt und gleichzeitig die endständige Funktion C nach beendeter Synthese freigesetzt wird, durch Einwirken einer starken Säure, wie insbesondere flüssige Fluorwasserstoffsäure.

17. Verfahren nach einem der Ansprüche 5 bis 16, dadurch <u>gekennzeichnet</u>, daß das nach Entfernen der Schutzgruppen erhaltene freie Peptid gereinigt wird durch Durchlaufen mindestens einer Säule mit einem geeigneten molekularen Harz und anschließende Reinigung durch Chromatographie in flüssiger Phase mit hoher Leistungsfähigkeit.

18. Mittel zum Schutz gegen Cholera und/oder gastroenteritische Infektionen, aus der Gruppe, die Seren und Vaccinen umfassen, die auf parenteralem, subcutanem, cutanem oder oralem Wege verabreichbar sind, dadurch <u>gekennzeichnet</u>, daß es ein synthetisches Peptid nach Anspruch 1 oder Anspruch 2 umfaßt.

19. Mittel zum Schutz nach Anspruch 18, dadurch <u>gekennzeichnet</u>, daß es das synthetische Peptid zusammen mit einer Untereinheit B des cholergen Toxins und/oder einer Untereinheit B des Toxins von Escherichia coli enthält.

20. Neues Arzneimittel, dadurch <u>gekennzeichnet</u>, daß es als aktiven Bestandteil enthält ein Peptid nach Anspruch 1 oder Anspruch 2, wobei das neue Arzneimittel geeignet ist zur Behandlung von Krankheiten, die mit einer Aktivierung von zyklischem A.M.P. einhergehen und insbesondere an eine Aktivierung des Sytems Adenylcyclase - zyklisches A.M.P. gebunden sind.

21. Neues Arzneimittel zur Behandlung von Cholera und gastroenteritischen Infektionen, dadurch <u>gekennzeichnet</u>, daß es als aktiven Bestandteil das synthetische Peptid nach Anspruch 1 oder Anspruch 2 enthält.

22. Diagnostisches Mittel für Cholera und/oder gastroenteritische Infektionen, dadurch <u>gekennzeichnet</u>, daß es eine Nukleotidsequenz gemäß einem der Ansprüche 3 und 4 enthält.

23. Diagnostisches Mittel für Cholera und/oder gastroenteritische Infektionen, dadurch <u>gekennzeichnet</u>, daß es ein synthetisches Peptid gemäß einem der Ansprüche 1 oder 2 enthält.

24. Diagnostisches Mittel nach Anspruch 23, dadurch <u>gekennzeichnet</u>, daß es im wesentlichen aus einem Serum besteht, das hergestellt wurde ausgehend von einem synthetischen Peptid gemäß einem der Ansprüche 1 oder 2.

25. Diagnostisches Mittel für die Anwesenheit von Escherichia coli-Toxinogenen, dadurch <u>gekennzeichnet</u>, daß es im wesentlichen aus einem Serum besteht, das aus Kaninchen erhalten wurde, die vorher durch Injektionen des synthetischen Peptids gemäß einem der Ansprüche 1 oder 2 behandelt wurden.

26. Diagnostischer Test auf die Anwesenheit von Escherichia coli-Toxinogenen, dadurch <u>gekennzeichnet</u>, daß man das diagnostische Mittel nach Anspruch 25 mit einem biologischen Medium, von dem angenommen wird, daß es die Toxinogenenkeime enthält, 16 bis 24 Stunden und vorzugsweise während 18 Stunden in Kontakt gebracht wird, wonach, falls eine Infektion vorliegt, eine Ausfällungsbande Antigen-Antikörper erscheint.

27. Diagnoseköfferchen, geeignet zur Verwendung zur Durchführung diagnostischer Untersuchungen, die die Bestimmung der Anwesenheit von pathogenen Keimen und insbesondere die Bestimmung der Anwesenheit von Escherichia coli-Toxinogenen nach Anspruch 26 ermöglichen, dadurch <u>gekennzeichnet</u>, daß es eine geeignete Menge eines diagnostischen Mittels für Cholera und/oder gastroenteritische Infektionen gemäß einem der Ansprüche 22 bis 25 und eine geeignete Menge eines Bezugsstandards, gebildet aus Antipeptid-Antikörpern gemäß einem der Ansprüche 1 oder 2 und insbesondere eine geeignete Menge Antikörper-Antipeptid 10-24, umfaßt.

**Patentansprüche** für den Vertragsstaat: AT

1. Verfahren zur Synthese eines synthetischen Peptids, das mindestens 5 bis 11 Aminosäuren enthält, die der Kette γ der Untereinheit A des cholergen Toxins und der Kette γ des LT-Toxins von Escherichia coli angehören, dadurch <u>gekennzeichnet</u>, daß das Peptid synthetisch hergestellt wird durch schrittweisen Aufbau der Peptidkette, wobei man von dem äußersten Ende C der Kette ausgeht, durch aufeinanderfolgende Fixierung der Aminosäuren, die diese bilden.

2. Verfahren zur Synthese nach Anspruch 1, dadurch <u>gekennzeichnet</u>, daß man ein Pentadecapeptid, das der nachstehenden Formel I entspricht:

10

```
Gln-Ser-Leu-Gly-Val-Lys-Phe-Leu-Asp-Glu-Tyr-Gln-Ser-Lys-Val  (I)
 10   11   12   13   14   15   16   17   18   19   20   21   22   23   24
```

wobei diese Formel der Sequenz 10-24 der Kette γ der Untereinheit A des cholergen Toxins entspricht und 5 bis 11 Aminosäuren aufweist, die der Kette γ der Untereinheit A des thermolabilen Toxins LT von Escherichia coli angehören, schrittweise ausgehend vom äußersten Ende C, d.h. ausgehend von dem Valin, durch aufeinanderfolgende Fixierung der Aminosäuren, die die Peptidkette bilden, aufbaut.

3. Syntheseverfahren nach Anspruch 1 oder Anspruch 2, dadurch gekennzeichnet, daß es in fester Phase in Anwesenheit eines geeigneten Kupplungsmittels durchgeführt wird.

4. Verfahren nach Anspruch 3, dadurch gekennzeichnet, daß die Synthese in fester Phase durchgeführt wird unter Verwendung eines festen Trägers, der aus einem Harz, insbesondere bevorzugt aus einem chlormethylierten Harz oder einem Phenolharz, besteht.

5. Verfahren nach einem der Ansprüche 1 bis 4, dadurch gekennzeichnet, daß die α-Aminfunktion der Aminosäuren, die nacheinander zur Bildung der gewünschten Peptidkette fixiert werden, temporär durch eine t-Butoxycarbonylgruppe (Boc) geschützt wird.

6. Verfahren nach einem der Ansprüche 1 bis 5, dadurch gekennzeichnet, daß die seitlichen Funktionen der nacheinander zur Bildung der gewünschten Peptidkette fixierten Aminosäuren bis zum Ende der Synthese durch Benzylgruppen geschützt werden.

7. Verfahren nach Anspruch 6, dadurch gekennzeichnet, daß die Carboxylgruppen der Asparginsäure und Glutaminsäure durch Benzylestergruppen geschützt werden.

8. Verfahren nach Anspruch 6, dadurch gekennzeichnet, daß die Hydroxylgruppe des Serins durch eine Benzylethergruppe geschützt wird.

9. Verfahren nach Anspruch 6, dadurch gekennzeichnet, daß das ε-Amin des Lysins durch eine Carbobenzyloxigruppe (BZ) geschützt wird.

10. Verfahren nach Anspruch 3, dadurch gekennzeichnet, daß das zur Durchführung des schrittweisen Aufbaus der Peptidkette verwendete Kupplungsmittel ein äquimolares Gemisch von Dicyclohexylcarbodiimid und 1-Hydroxybenzotriazol in einem geeigneten Lösungsmittel, wie inbesondere Dimethylformamid oder Methylenchlorid ist.

11. Verfahren nach Anspruch 5, dadurch gekennzeichnet, daß die Schutzgruppe Boc der α-Aminfunktion der nacheinander fixierten Aminosäuren durch Acidolyse abgespalten wird.

12. Verfahren nach einem der Ansprüche 6 bis 9, dadurch gekennzeichnet, daß die Schutzgruppen der seitlichen Funktionen der nacheinander zur Bildung der gewünschten Peptidkette fixierten Aminosäuren entfernt und gleichzeitig die endständige Funktion C nach beendeter Synthese freigesetzt wird, durch Einwirken einer starken Säure, wie insbesondere flüssige Fluorwasserstoffsäure.

13. Verfahren nach einem der Ansprüche 1 bis 12, dadurch gekennzeichnet, daß das nach Entfernen der Schutzgruppen erhaltene freie Peptid gereinigt wird durch Durchlaufen mindestens einer Säule mit einem geeigneten molekularen Harz und anschließende Reinigung durch Chromatographie in flüssiger Phase mit hoher Leistungsfähigkeit.

14. Verfahren nach Anspruch 1, dadurch gekennzeichnet, daß man eine Nukleotidsequenz, die mindestens 5 Aminosäuren des synthetischen Peptids nach Anspruch 1 entspricht, aufbaut.

15. Verfahren nach Anspruch 2, dadurch gekennzeichnet, daß man eine Nukleotidsequenz, die 15 Aminosäuren des Pentadecapeptids nach Anspruch 2 entspricht, aufbaut.

**Claims** for the contracting States: BE, CH, DE, FR, GB, IT, LI, LU, NL, SE. 1. Synthetic peptide characterized in that reproduces at least 5 of the 11 amino acids common to the γ chain of the sub-unit A of the cholera toxin and to the γ chain of the Escherichia coli LT toxin.

2. Peptide according to claim 1, characterized in that it is constituted by a pentadecapeptide which corresponds to the following formula 1:

0 139 555

Gln-Ser-Leu-Gly-Val-Lys-Phe-Leu-Asp-Glu-Tyr-Gln-Ser-Lys-Val

10  11  12  13  14  15  16  17  18  19  20  21  22  23  24

which formula corresponds to the sequence 10-24 of the γ chain of the sub-unit A of the cholera toxin and has 5 of the 11 amino acids common with the γ chain of the sub-unit A of the thermolabile LT toxin of Escherichia coli.

3. Nucleotide sequence characterized in that it corresponds to the minimum 5 amino acids of the synthetic peptide according to claim 1.

4. Nucleotide sequence characterized in that it corresponds to the fifteen amino acids of the pentadecapeptide according to claim 2.

5. Process of synthesis of a peptide according to claim 1, characterized in that said peptide is synthesized by construction of the peptide chain step by step, starting from the C terminal end of said chain, by successive fixing of the amino acids which constitute the latter.

6. Process of synthesis of the pentadecapeptide of formula I according to claim 2, characterized in that said pentadecapeptide is synthesized by construction of the peptide chain step by step, starting from the C terminal end, that is to say starting from valine, by successive fixation of the amino acids which constitute the peptide chain.

7. Process of synthesis according to claim 5 or claim 6, characterized in that it is carried out in the solid phase, in the presence of a suitable coupling agent.

8. Process according to claim 7, characterized in that the synthesis in the solid phase is carried out by using a solid support constituted by a resin, preferably a chloromethylated resin or a phenolic resin, particularly.

9. Process according to any one of claims 5 to 8, characterized in that the α -amino function of the amino acids successively fixed to form the desired peptide chain, is protected temporarily by a t-Butoxycarbonyl group (Boc).

10. Process according to any one of claims 5 to 9, characterized in that the lateral functions of the amino acids successively fixed to form the desired peptide chain, are protected until the end of the synthesis by benzyl groups.

11. Process according to claim 10, characterized in that the carboxyl groups of the aspartic and glutamic acids are protected by benzyl ester groups.

12. Process according to claim 10, characterized in that the hydroxyl group of the serine is protected by a benzylether group.

13. Process according to claim 10, characterized in that the ε amino of the lysine is protected by a carbobenzyloxy group (GBZ).

14. Process according to claim 7, characterized in that the coupling agent employed to effect the step by step construction of the peptide chain is an equimolecular mixture of dicyclohexylcarbodiimide and 1-hydroxybenzotriazole in a suitable solvent, such as, particularly, dimethylformamide or methylene chloride.

15. Process according to claim 9, characterized in that the protective group Boc of the α-amino function of the successively fixed amino acids is removed by acidolysis.

16. Process according to any one of claims 10 to 13, characterized in that the protective groups of the amino acids successively fixed to form the desired peptide chain, are eliminated and the C terminal function is freed, simultaneously once the synthesis is terminated, by the action of a strong acid, such as, particularly, liquid hydrofluoric acid.

17. Process according to any one of claims 5 to 16, characterized in that the free peptide obtained after the elimination of the protective group, is purified by passage over at least one column of a suitable molecular sieve, then purification by high performance liquid phase chromatography.

18. Protective against cholera and/or infectious gastroenterites, taken from the group which comprises the serums and vaccines administrable by parenteral, subcutaneous, cutaneous, or oral routes, characterized in that it comprises a synthetic peptide according to claim 1 or claim 2.

19. Protective agent according to claim 18, characterized in that comprises said synthetic peptide associated with a sub-unit B of cholera toxin and/or with a sub-unit B of Escherichia coli toxin.

20. Novel medicament characterized in that it comprises, as active constituent, a peptide according to claim 1 or claim 2, said novel medicament being adapted to treat diseases associated with activation of the cyclic AMP and more particularly connected with activation of the adenylcyclase - cyclic AMP system.

21. Novel medicament for the treatment of cholera and infectious gastro-enterites, characterized in that it comprises as active constituent, the synthetic peptide according to claim 1 or claim 2.

22. Diagnostic agent for cholera and/or infectious gastro-enterites, characterized in that it comprises a sequence of nucleotides according to any one of claims 3 and 4.

23. Diagnostic agent for cholera and/or infectious gastro-enterites, characterized in that it comprises a synthetic peptide according to any one of claims 1 or 2.

24. Diagnostic agent according to claim 23, characterized in that it is essentially constituted by a serum

12

prepared from the synthetic peptide according to any one of claims 1 or 2.

25. Diagnostic agent for the presence of toxinogenic Escherichia coli, characterized in that it is essentially constituted by serum taken from rabbits previously treated by injections of the synthetic peptide according to any one of claims 1 or 2.

26. Diagnostic test for the presence of toxinogenic Escherichia coli, characterized in that the diagnostic agent according to claim 25 is contacted with a biological medium assumed contaminated by the above-said toxinogenic germs, for 16 to 24 hours and preferably for 18 hours, at the end of which there appears, if the medium is contaminated, a band of antigen-antibody precipitate.

27. Diagnostic kit ready for use for carrying out diagnostic tests enabling the detection of the presence of pathogenic germs and particularly for the detection of the presence of toxinogenic Escherichia coli, according to claim 26, characterized in that it comprises a suitable amount of a diagnostic agent for cholera and/or infectious gastro-enterites according to any one of claims 22 to 25 and a suitable amount of a reference standard constituted by antipeptide antibodies according to any one of claims 1 or 2, and particularly by a suitable amount of antipeptide 10-24 antibodies.

**Claims** for the contracting State: AT.

1. Process of synthesis of a synthetic peptide which reproduces at least 5 of the 11 amino acids common to the γ chain of the sub-unit A of the cholera toxin and to the γ chain of the Escherichia coli LT toxin, characterized in that said peptide is synthesized by construction of the peptide chain step by step, starting from the C terminal end of said chain, by successive fixation of the amino acids which constitute the latter.

2. Process according to claim 1, characterized in that a pentadecapeptide is obtained which corresponds to formula 1

Gln-Ser-Leu-Gly-Val-Lys-Phe-Leu-Asp-Glu-Tyr-Gln-Ser-Lys-Val

10  11  12  13  14  15  16  17  18  19  20  21  22  23  24

which formula corresonds to the sequence 10-24 of the γ chain of the sub-unit A of the choleric toxin and has 5 of 11 amino acids commom with the γ chain of the sub-unit A of the Escherichia coli LT thermolabile toxin, by construction of the peptide chain step by step, starting from the C terminal end, that is to say, starting from valine, by successive fixation of the amino acids which constitute the peptide chain.

3. Process of synthesis according to claim 1 or 2, characterized in that it is carried out in the solid phase, in the presence of a suitable coupling agent.

4. Process according to claim 3 characterized in that the synthesis in the solid pnase is carried out by using a solid support constituted by a resin, preferably a chloromethylated resin or a phenolic resin, particularly.

5. Process according to any one of claims 1 to 4 characterized in that the α -amino function of the amino acids successively fixed to form the desired peptide chain, is protected temporarily by a t-Butoxycarbonyl group (Boc).

6. Process according to any one of claims 1 to 5 characterized in that the lateral functions of the amino acida successively fixed to form the desired peptide chain, are protected until the end of the synthesis by benzyl groups.

7. Process according to claim 6 characterized in that the carboxyl groups of the aspartic and glutamic acids are protected by benzyl ester groups.

8. Process according to claim 6 characterized in that the hydroxyl group of the serine is protected by a benzylether group.

9. Process according to claim 6 characterized in that the ε amino of the lysine is protected by a carbobenzyloxy group (CBZ).

10. Process according to claim 3 characterized in that the coupling agent employed to effect the step by step construction of the peptide chain is an equimolecular mixture of dicyclohexylcarbodiimide and 1-hydroxybenzotriazole in a suitable solvent, such as, particularly, dimethylformamide or methylene chloride.

11. Process according to claim 5 characterized in that the protective group Boc of the α-amino function of the successivaly fixed amino acids is ramoved by acidolysis.

12. Process according to any one of claims 6 to 9 characterized in that the protective groups of the amino acids successively fixed to form the desired peptide chain, are eliminated and the C terminal function is freed, aimultaneously once the synthesis ia terminated, by the action of a strong acid, such as, particularly, liquid hydrofluoric acid.

13. Process according to any one of claims 1 to 12 characterized in that the free peptide obtained after the elimination of the protective group, is purified by passage over at least one column of a suitable molecular sieve, then purification by high performance liquid phase chromatography.

14. Process according to claim 1, characterized in that five minimum amino acids of the aynthetic peptide are obtained.

15. Process according to claim 2, characterized in that a nucleotide sequence is obtained corresponding to the fifteen amino acida of the pentadecapeptide.